# FASCICULE DE BREVET EUROPEEN

(11) **EP 0 863 857 B1**
(45) Date de publication et mention de la délivrance du brevet: **09.10.2002**
(21) Numéro de dépôt: 96939969.0
(22) Date de dépôt: 22.11.1996
(51) Int. Cl.: C07B 41/02, C07C 29/62, C07C 29/38, C07C 33/50, C07C 45/45, C07C 49/80, C07C 219/06, C07F 7/18, C07C 305/06, C07C 319/14, C07C 323/03

(54) **PROCEDE UTILE POUR LA PERFLUOROALCOYLATION ET REACTIF POUR METTRE EN OEUVRE CE PROCEDE**
PERFLUOROALKYLIERUNGSVERFAHREN UND REAGENZ DESSELBEN
PERFLUOROALKYLATION METHOD AND REAGENT THEREFOR

(30) Priorité: 23.11.1995 FR 9513996; 15.11.1996 FR 9614134
(43) Date de publication de la demande: 16.09.1998
(73) Titulaire: RHODIA CHIMIE, 92408 Courbevoie Cédex (FR)
(72) Inventeur: ROQUES, Nicolas, F-69008 Lyon (FR); RUSSELL, James, F-30340 Rousson (FR)
(74) Mandataire: Ricalens, François
(86) Numéro de dépôt international: FR9601854
(87) Numéro de publication internationale: WO97019038

(56) Documents cités:
- JOURNAL OF ORGANIC CHEMISTRY, vol. 56, no. 1, 4 Janvier 1991, WASHINGTON, DC, US, pages 2-4, XP002009863 T. SHONO, ET AL.: "Eletctroorganic chemistry. 130. A novel trifluoromethylation of aldehydes and ketones promoted by an electrogenerated base"

## Description

La présente invention a pour objet un procédé utile pour la perfluoroalcoylation et ainsi qu'un réactif pour mettre en oeuvre ce procédé. Elle concerne plus particulièrement un réactif et un procédé pour greffer un groupement difluorométhyle substitué sur un composé comportant au moins une fonction électrophile. Elle concerne plus particulièrement une technique pour perfluoroalcoyler différents composés par des réactions de substitution nucléophile ou d'addition typiquement réalisées par des dérivés organométalliques.

Les techniques de perfluoroalcoylation, ou les techniques équivalentes, utilisent en général des dérivés du type iodure de perfluoroalcoyle, en présence de zinc. Cette technique est donc coûteuse, tout en nécessitant des installations de traitement des rejets métalliques qu'il convient de traiter car le zinc est un polluant important des cours d'eau.

Les autres techniques, où le radical perfluoroalcoyle ne forme pas un intermédiaire réactif stabilisé du type organo-métallique, sont en général difficiles à mettre en oeuvre en raison de la très faible stabilité des anions perfluorés libres dans les milieux réactionnels. Ces derniers conduisent en général à des produits du type carbène, lesquels lorsqu'ils réagissent ont perdu un de leurs substituants.

C'est pourquoi un des buts de la présente invention est de fournir un réactif qui permette une perfluoroalcoylation selon un mécanisme du type faisant intervenir carbanion, sans faire appel à des organométalliques de métaux de transition comme le zinc.

On a souvent cherché à utiliser comme source de radicaux perfluoroalcoyle, plus généralement de radicaux trifluorométhyle, des acides perfluorocarboxyliques, en mettant en oeuvre des réactions de décomposition visant à éliminer le fragment carboxylique desdits acides en libérant du bioxyde de carbone. Toutefois, les succès qui avaient été obtenus étaient très mitigés et utilisaient des systèmes catalytiques particulièrement compliqués. Les radicaux perfluoroalcoyle ou leurs équivalents engendrés par la décomposition desdits acides perfluorocarboxyliques étaient en outre instables dans le milieu réactionnel et nécessitaient l'emploi d'agents stabilisants.

Plus récemment SHONO dans un article intitulé "a novel trifluorométhylation of aldehydes and ketones promoted by an electrogenerated base " et publié dans J. Org. Chem. 1991, 56, 2-4 a essayé de réaliser des réactions de perfluorométhylation à partir de fluoroforme et montré qu'il était très difficile d'obtenir des ds'obtenir des résultats positifs en l'absence de la base constituée de l'anion pyrolidonyle associé à un cation ammonium quaternaire et ce à la condition expresse que cette base fût engendrée par électrolyse.

Au cours de cette étude comparative, en prenant comme réaction test la trifluorométhylation du benzaldéhyde selon la technique dite de Barbier (qui sera détaillée ci après), cet auteur concluait que les résultats obtenus à partir d'autres bases donnait des rendements nuls ou médiocres et que les réactions parasites et notamment celle de Cannizaro (dismutation du benzaldéhyde en acide benzoïque et en alcool benzylique), étaient majoritaires [mais les modes opératoires relatifs aux bases usuelles (tertiobutylate de potassium, hydrure de sodium,.....) n'y sont pas décrits].

Toutefois les techniques utilisant les bases électroengendrées décrites par cet auteur nécessitent d'une part un appareillage complexe et d'autre part un tour de main tel qu'elles sont délicates à reproduire et extrêmement difficiles à extrapoler à des échelles industrielles. Enfin l'utilisation des ammoniums quaternaires qui sont très hygroscopiques implique beaucoup de minutie.

La présente invention se propose d'obvier aux inconvénients des procédés existants en fournissant un réactif non nocif pour l'environnement et capable de conduire aux produits désirés avec un rendement satisfaisant.
Ces buts et d'autres qui apparaîtront par la suite sont atteints au moyen d'un procédé comporte une étape de mise en contact d'un matériau de formule RfH et d'une base, y compris une espèce susceptible d'engendrer une base, avec un substrat porteur d'au moins une fonction électrophile par addition dans un milieu polaire, caractérisé par le fait que la réaction est menée de manière à introduire en premier dans le milieu, soit le matériau RfH, soit la base et enfin le substrat en dernier.
Comme espèce susceptible d'engendrer une base forte en présence d'un composé à hydrogène mobile tel que par exemple le toluène, on peut citer comme exemple d'une telle espèce les métaux alcalins, voire alcalino terreux.

Les conditions ci -après sont préférées pour les substrats basosensibles :
◆ conduire l'addition, puis éventuellement à poursuivre la réaction après l'addition de manière que d'une part au moins 90 % de l'addition du dernier réactif soit réalisée et que d'autre part le mélange réactionnel ait été maintenu au moins 1/2 heure (y compris la durée de l'addition) à une température au plus égale à -20°C, avantageusement à -30°C ;
◆ de remplir au moins une des, de préférence les deux, conditions ci-après :
   ⇒ la teneur en eau est limitée à une valeur au plus égale à 200 ppm (deux chiffres significatifs), avantageusement à 100 ppm (deux chiffres significatifs),.de préférence à 50 ppm (un chiffre significatif) ;
   ⇒ Soit la quantité de base est au plus égale à 1,3 fois la quantité stoechiométrique par rapport au substrat avec une température au plus égalé à 0°C ou bien la base est au plus égale à 1,1 fois la quantité stoechiométrique avec une température au plus égale à 20°C ;

Il convient de signaler que les conditions ci dessus sont favorables même quand la substrat n'est pas basosensible. Toutefois il peut parfois être intéressant de réaliser des réactions en s'écartant quelque peut des conditions optimales lorsque les conditions économique le sont. (Cf. les domaines préféré d'anhydricité).

Dans la présente description, Par H-Rf on entend des radicaux de formule :

H-(CX₂)ₚ-GEA (II)

où les X semblables ou différents représentent, un fluor ou un radical de formule CₙF₂ₙ₊₁ avec n entier au plus égal à 5 de préférence à 2, voire un chlore ;
où p représente un entier au moins égal à 1 et au plus égal à 2 ;
où GEA représente un groupe électroattracteur dont les éventuelles fonctions sont inertes dans les conditions de la réaction, avantageusement fluor ou un reste perfluoré de formule CₙF₂ₙ₊₁ avec n entier au plus égal à 8, avantageusement à 5 ;
avec la condition que X ne peut être qu'une fois chlore sur un même carbone. Le cas où le carbone portant l'atome d'hydrogène, présente deux X différent de chlore est particulièrement intéressant.

II est également souhaitable que parmi les X et GEA au moins un avantageusement 2 soient des atomes (de chlore ou de fluor).

Le nombre total de carbone de Rf est avantageusement compris entre 1 et 15, de préférence entre 1 et 10.

Dans le matériau RfH du réactif de l'invention, l'entité GEA qui exerce un effet électroattracteur sur l'atome de carbone difluoré est de préférence choisie parmi les groupes fonctionnels dont la constante de Hammett σₚ est au moins égale à 0,1. Il est en outre préférable que la composante inductive de σₚ, σᵢ, soit au moins égale à 0,2, avantageusement à 0,3. A cet égard, on se référera à l'ouvrage de March, "Advanced Organic Chemistry", troisième édition, John Wiley and Son, pages 242 à 250, et notamment au tableau 4 de cette section.

Plus particulièrement, l'entité électroattractrice peut être choisie parmi les atomes d'halogène, de préférence légers [notamment de chlore et de fluor]. Le matériau RfH correspondant est, quand p est égal à 1, un Halogénoforme.
GEA peut également être avantageusement choisie parmi les groupements nitriles, carbonylés, sulfonés et perfluoroalcoylés.
Des matériaux préférés de formule RfH de ce type qui peuvent être utilisés répondent à la formule R - G - CF.₂ - H
- où G représente un groupement divalent de formule -Z-G'- dans lequel
   ◆ le divalent Z représente un liaison simple, un atome de chalcogène, un reste divalent -Y(R')- où R' est un radical hydrocarboné d'au plus dix atomes, avantageusement d'au plus six atomes avantageusement d'au plus deux atomes de carbone et où Y est un atome métalloïde de la colonne V (azote, phosphore,....) ;
   ◆ G' représente >C=O, >S=O, -SO₂-, ou - (CF_{·2}) ₙ- avec n entier supérieur ou égal à 1 ;
- et où R représente un résidu organique ou minéral indifférent, de préférence un radical organique tel que aryle, akoyle, y compris aralcoyle, éventuellement substitué. R peut également représenter un support solide minéral ou organique, tel qu'une résine ;
- ou bien l'ensemble R - G représente un groupe nitrile, ester, amide (avantageusement non porteur d'hydrogène), y compris sulfamide.

Dans le cas où G représente un groupe perfluoroalcoylène - (CF.₂)ₙ -, n est avantageusement compris entre 1 et 10, de préférence entre 1 et 5. Toujours dans ce cas, R peut également représenter un atome d'halogène, notamment le fluor.

Ainsi, selon une variante avantageuse de la présente invention, ledit matériau de formule R_{f}H répond à la formule II où GEA représente un groupe électroattracteur de formule III :

R-CₙX'₂ₙ- (III)

Où n est un entier au plus égal à 5,
où R est choisi parmi l'hydrogène, un radical hydrocarboné, tel que les aryles et les alcoyles de 1 à 10 atomes de carbone, et les halogènes légers (chlore ou fluor, avantageusement fluor) ;
où les X', semblables ou différents représentent un halogène léger (chlore ou fluor, avantageusement fluor) ou un radical de formule CₘF₂ₘ₊₁ avec m entier au plus égal à 5 de préférence à 2.

Lorsque R représente un hydrogène, la réaction est plus complexe, ledit matériau pouvant réagir par plusieurs bouts ; et les rapports entre les réactifs doivent tenir compte de cette réactivité dans la stoechiométrie. Cette polyvalence des matériaux peut être un inconvénient et de ce fait la valeur hydrogène pour R n'est pas communément souhaitable.

Il est souhaitable que au moins trois quarts, avantageusement au moins neuf dixièmes, de préférence la totalité, éventuellement moins un, des X et des X' soient des fluors ou des perfluoroalcoyles (stricto sensu soit de formule générale de type C_{ν}F_{2ν+1})

Avantageusement, l'acide associé de ladite base présente un pKa au moins égal à 15.

Toutefois pour obtenir de bons résultats il faut, soit que ledit substrat porteur d'au moins une fonction électrophile soit très favorable (aldéhyde ou cétone ne possédant pas d'hydrogène acide en alpha), soit que l'acide associé de ladite base présente un pKa au moins égal à 20, avantageusement à 25, de préférence à 30.

En outre, surtout quand la base est dans la zone basse des valeurs ci-dessus, il est souhaitable que ladite base présente un acide associé volatil dans les conditions de la réaction.

Avantageusement, ledit milieu polaire et anhydre est tel que l'acide le plus fort présent dans le milieu compte non tenu du matériau RfH et du substrat présente un pKa au moins égal à 25, avantageusement à 30, de préférence à 35.

Plus le milieu sera aprotique, c'est-à-dire que sa teneur en protons libérables dans le réactif sera faible, moins il y aura de risque de réaction parasite et meilleur sera le rendement.

Ainsi, il est préférable que, dans le réactif, la teneur en atomes d'hydrogène labites soit au plus égale à 1/3, avantageusement à 1/4, de préférence à 10 % (en moles), par rapport à la teneur initiale en celui de ladite base ou dudit matériau qui n'est pas en excès.

Cet effet est particulièrement important lorsque la réaction est menée à une température supérieure à environ 240 K (dans la présente description le terme "environ" est employé pour mettre en exergue le fait que, lorsque le, ou les, chiffres les plus à droite d'un nombre sont des zéros, ces zéros sont des zéros de position et non des chiffres significatifs, sauf bien entendu s'il en est précisé autrement).

La principale impureté, porteuse d'atomes d'hydrogène labiles, est en général l'eau qui est susceptible de libérer jusqu'à deux atomes d'hydrogène par molécule.

C'est pourquoi, ledit milieu polaire est avantageusement anhydre, y compris substrat et le matériau RfH, c'est-à-dire qu'il présente une quantité molaire en eau inférieure au tiers de la quantité de base introduite, avantageusement au quart, de préférence au dixième. Cette contrainte d'anhydricité est peu importante pour des procédés où la réaction est menée à des températures inférieures à 240°K (deux chiffres significatifs).

D'une manière générale il est préférable d'utiliser des réactifs et des solvants soigneusement déshydratés, de manière que la teneur pondérale en eau du réactif soit au plus égale à 1 pour 100, avantageusement 1 pour 1000, de préférence à 1 pour 10.000 par rapport à la masse totale du réactif.

Par ailleurs, il a pu être montré que d'autres éléments, à savoir les éléments de transition ayant deux états de valence stable, tels le cuivre, pouvaient n'être pas fastes, voire pouvaient être néfastes.

Bien que ce réactif selon l'invention ne nécessite pas de catalyseur, de tels éléments métalliques peuvent être présents en tant qu'impuretés apportées notamment par le solvant.

Ainsi, il est préférable que la teneur molaire en ces éléments soit inférieure à 1000, avantageusement à 100, de préférence à 10 ppm par rapport à la teneur initiale en ledit matériau RfH.

Egalement, bien qu'il ait été de nombreuses fois préconisé d'utiliser avec les agents de perfluoroalcoylation, des éléments de la colonne VIII de la classification périodique des éléments, pour favoriser certains substrats et favoriser certains types de réaction, cela ne s'est pas révélé particulièrement faste pour la réaction visée ci-dessus. C'est pourquoi il est préférable d'utiliser des réactifs ne contenant pas de métaux de la colonne VIII, notamment des métaux de la mine du platine qui est le groupe constitué du platine, de l'osmium, de l'iridium, du palladium, du rhodium et du ruthénium.

Dans la présente description, il est fait référence au supplément au bulletin de la Société Chimique de France numéro 1, janvier 1966, où une classification périodique des éléments a été publiée.

Ainsi il est préférable que la teneur en métaux de la mine du platine, voire en métaux de la colonne VIII, soit inférieure à 100 ppm, avantageusement à 10 ppm, de préférence 1 ppm. Ces valeurs s'entendent par rapport à la base de départ et sont exprimées en moles.

D'une manière plus générale et plus empirique, on peut indiquer que ces deux catégories de métaux, à savoir les éléments de transition à deux états de valence et les éléments de la colonne VIII, doivent être présents dans le réactif à un niveau de concentration globale au plus égal à 1000 ppm molaires, de préférence à 10 ppm molaires.

On notera que les différents métaux présents à un tel niveau de concentration globale sont en quantité extrêmement faible et, à cet égard, ils ne jouent aucun rôle catalytique. Leur présence n'améliore pas la cinétique de la réaction, voire lui est néfaste lorsqu'ils sont présents en trop grande quantité.

L'utilisation, en plus des composants de réactifs précités, de fluorure de métal alcalin ou de fluorure de phosphonium quaternaire [voire d'ammonium quaternaire si l'on respecte les contraintes que ce type de composé engendre], habituellement présents dans les systèmes réactifs utilisant des carboxylates fluorés, ne s'est pas révélée néfaste, mais elle s'est révélée en général de peu d'intérêt, notamment en raison du fait qu'elle produit des effluents salins difficiles à traiter. C'est la raison pour laquelle il est préférable de limiter leur teneur, en particulier leur teneur initiale. Ainsi il est préférable que la teneur en fluorure, que l'on qualifie de ionique, c'est-à-dire susceptible d'être ionisé dans le milieu polarisant du réactif, soit au plus égale à la concentration molaire initiale en ledit matériau RfH, avantageusement à la moitié, de préférence au quart.

Ledit milieu polaire peut comporter des solvants.

Même s'il s'agit d'une tautologie, il faut rappeler que le solvant (qui peut comprendre plusieurs constituants) doit être liquide aux températures d'utilisation.

On peut notamment indiquer qu'il est souhaitable que ledit solvant présente un point de congélation commençante (apparition d'une phase solide issue du solvant) au plus égal à 10° C, avantageusement à 0°C, de préférence à -10° C. Dans le cas ou l'on veut pouvoir travailler avec une plus grande tolérance en H⁺ et/ou en eau (cas notamment où l'on voudrait utiliser des ammoniums quaternaires) il est conseillé de choisir un solvant dont le point de congélation commençante (apparition d'une phase solide issue du solvant) est au plus égal à - 30° C.

Ainsi les solvants eux-mêmes peuvent être constitués par des mélanges. Notamment ces mélanges peuvent comporter des solvants polaires et des solvants non, ou peu, polaires que l'on qualifiera ci-après de diluant.

Ainsi qu'on l'a mentionné ci-dessus, le solvant joue un rôle important dans la présente invention et doit être aprotique, et avantageusement polaire et comporter très peu d'impuretés porteuses d'hydrogène acide.

Il est ainsi préférable que le solvant aprotique polaire utilisable ait un moment dipolaire significatif. Ainsi, sa constante diélectrique relative ε est avantageusement au moins égale à environ 5. De préférence l'e est inférieur ou égal à environ 50 (dans la présente description le terme "environ" est employé pour mettre en exergue le fait que, lorsque le, ou les, chiffres les plus à droite d'un nombre sont des zéros, ces zéros sont des zéros de position et non des chiffres significatifs, sauf bien entendu s'il en est précisé autrement) et supérieur ou égal à 5.

On préfère en outre que les solvants polaires de l'invention soient susceptibles de bien solvater les cations, ce qui peut être codifié par l'indice donneur D de ces solvants. II est ainsi préférable que l'indice donneur D de ces solvants soit compris entre 10 et 30, avantageusement entre 20 et 30. Ledit indice donneur correspond au ΔH (variation d'Enthalpie), exprimé en kilocalorie, de l'association dudit solvant aprotique polaire avec le pentachlorure d'antimoine. Plus précisément dans l'ouvrage de Christian REICHARDT, [Solvents and Solvent Effects in Organic Chemistry - VCH p.19 (1988)], on trouve la définition de l'indice donneur ("donor number" en anglo-saxon) qui est défini comme le négatif (-ΔH) de l'enthalpie (Kcal/mol) de l'interaction entre le solvant et le pentachlorure d'antimoine, dans une solution diluée de dichtorométhane.

Un des avantages des cryptants est de permettre de s'affranchir au moins partiellement des solvants à fort indice donneur.

Selon la présente invention, il est préférable que le réactif ne présente pas d'hydrogène acide sur le ou les solvants polaires qu'il comporte. En particulier lorsque le caractère polaire du ou des solvants est obtenu par la présence de groupes électroattracteurs, Il est souhaitable qu'il n'y ait pas d'hydrogène en alpha de la fonction électroattractrice.

De manière plus générale, il est préférable que le pKa correspondant à la première acidité du solvant soit au moins égal à environ 20 ("environ" soulignant que seul le premier chiffre est significatif), avantageusement au moins égal à 25, de préférence compris entre 25 et 35.

Il est préférable que la base soit au moins partiellement, de préférence complètement, soluble dans le milieu constituant le réactif. Il en va de même avec le matériau de formule RfH.

Les solvants polaires donnant de bons résultats peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). On peut citer par exemple les dérivés de pyrrolidone, tels que la N-méthylpyrrolidone, ou encore le N,N-diméthylformamide, ou le N,N-diméthyllacétamide.

Une autre catégorie particulièrement intéressante de solvants polaires est constituée par les éthers, qu'il soient symétriques ou non symétriques, qu'ils soient ouverts ou cycliques. Dans la catégorie des éthers doivent être incorporés les différents dérivés des éthers de glycol tels que les différents glymes, le diglyme par exemple. On peut également citer une autre catégorie les dérivés sulfoxygénés tels que les sulfoxydes et notamment le DMSO.
Ainsi les solvants polaires les plus adéquats, en raison de leurs prix et de leurs propriétés, sont avantageusement choisis parmi les éthers, notamment cycliques, tels le THF ou polyfonctionnels tels les glymes, ceux des amides qui, tels le DMF
ou les DAAU (N,N'DiAlcoylAlcoylèneUrée) tels que la DMEU (N,N'DiMéthylEthylène-Urée) ou la DMPU (N,N'DiMéthylPropylèneUrée) n'ont pas d'hydrogène acide et les hétérocycles à caractère basique tel la pyridine et leurs mélanges.

Outre les solvants polaires proprement dits qui jouent un rôle de solvatation lequel est corrélé avec l'indice donneur, le solvant peut comprendre des diluants ne possédant pas cette propriété. Parmi les diluants, on peut citer les hydrocarbures aliphatiques ou aromatiques, tels les alcanes ou les dérivés aryliques. Mention doit être faite des arylméthanes qui peuvent à la fois servir de diluant, (car inerte dans les conditions de la réaction) et de sources de base lorsque cette demière est faite préalablement in situ.

Les contres cations susceptibles de potentialiser la base pour faire réagir le matériau de formule RfH sont avantageusement volumineux. Ainsi, on préfère des sels alcalins, avantageusement ceux oùs le métal alcalin est choisi parmi le sodium, le potassium, le rubidium, le césium et le francium. De préférence, ledit métal est d'une période dont le rang est au moins égal à celle du sodium, avantageusement à celle du potassium. On préfère également des sels de phosphonium quaternaire voire d'ammonium quatemaire si l'on respecte les contraintes que ce type de composé engendre.

II est également possible d'améliorer la réaction, notamment lorsque le solvant est éther ou en contient, en utilisant des cations qui sont, soit naturellement volumineux comme les phosphoniums quaternaires [voire d'ammonium quaternaire si l'on respecte les contraintes que ce type de composé engendre], ou rendus volumineux par l'addition d'agents chélatants ou de préférence cryptants, tels que par exemple les éthers couronne ou les dérivés qui sont à la fois aminés et oxygénés. Quoiqu'il présente l'inconvénient souvent dirimant d'être très hygroscopique, les cations (à fonction) ammonium quaternaire peuvent être utilisés, à condition de prendre des précautions drastiques.

Ledit substrat peut être choisi parmi les composés hydrocarbonés halogénés ou pseudohalogénés, notamment les halogénures ou pseudohalogénures d'alcoyle, d'aryle ou d'aralcoyle, les dérivés halogénés de composés organiques du silicium, notamment les halogénures de silane ou de siloxane, les dérivés halogénés de composés organiques du soufre, notamment les halogénures de sulfényle, de sulfinyle ou de sulfonyle, où l'atome d'halogène ou le groupement pseudohalogène est substitué au cours de la réaction par un groupement difluorométhyle substitué, ou les composés de type thiocyanate où le groupe cyano est substitué au cours de la réaction par un groupement difluorométhyle substitué. Lorsque l'intermédiaire tétraédrique est présent (technique Grignard en présence d'un carbonyle additionnable de manière stable : amide) et lorsque le substrat est tel que la réaction de fluoroalcoylation ne peut pas passer (ou passe difficilement) par un intermédiaire d'addition, ce type de substrat peut évoluer, notamment donner un dérivé stable de l'intermédiaire tétraédrique, c'est-à-dire que la Substitution Nucléophile se fait par le dérivé tétraédrique et non par le Rf⁻.

Dans les composés ci-dessus, l'atome d'halogène peut être choisi parmi les atomes d'iode, de brome, de chlore et de fluor. Un groupement "pseudohalogène" est un groupe qui, partant, sous forme anionique, présente un acide associé dont le pKa est inférieur à 4, de préférence à 3, en particulier à 0.

On préfère les groupes dont l'acide associé présente une acidité (mesurée par la constante de Hammett) au moins égale à celle de l'acide acétique, avantageusement à celle des acides sulfoniques, ou des acides α-trihalogénés. Un des pseudohalogènes typiques est un groupe perfluoroalcanesulfonyloxyle qui libère un perfluoroalcanesulfonate. Des groupes pseudohalogènes préférés peuvent être choisis parmi les groupes qui donnent un groupe partant appartenant aux sulfonates dont les paradigmes, car les plus utilisés, sont les tosylates (anion p-toluènesulfonyloxyle, ou p-toluènesulfonyloxylate), mésylate (méthylsulfonyloxylate), triflate (trifiuorométhylsulfonyloxylate) ou bien les carboxylates α-polyhalogénés dont l'un des paradigmes est le trifluoroacétate. On peut même considérer le groupe acyloxylate (c'est-à-dire carboxylate, par exemple acétate) comme un tel groupe partant.

Au cours de l'étude qui a mené à la présente invention, il a été toutefois montré qu'il est souhaitable que ledit substrat soit porteur d'au moins une fonction électrophile par addition. En d'autres termes que la réaction se fasse, en tout cas à titre transitoire, par addition sur une fonction présentant une double liaison (naturellement y compris celle de type donneur accepteur) ou un doublet appartenant à un métalloïde de période de rang au moins égale à 3.

Ainsi, selon une mise en oeuvre particulièrement avantageuse de la présente invention, une telle fonction électrophile par addition, est choisie parmi les fonctions carbonyle, thiocarbonyle(>C=S), éventuellement conjuguées avec une ou plusieurs liaisons de type éthylénique, les chalcogènures (dont le chalcogène est de rang atomique au moins égal à celui du soufre) portant un bon groupe partant (voir ci-dessus) et notamment les dichalcogènures (dont les chalcogènes sont des rang atomique au moins égal à celui du soufre).

Ainsi, le réactif réagit également avantageusement sur un composé choisi parmi les composés carbonylés de type cétone, aldéhyde, ester activé (voire halogénure d'acide) ou ester non activé, en réalisant une addition sur la fonction carbonyle. Le produit de la réaction est un alcool(ate) dont l'atome de carbone porteur de la fonction hydroxyle est substitué par un groupement difluorométhyle substitué. Ces alcooiates transitoires, après hydrolyse (en général acide), donnent le composé de substitution ou d'addition. Le cas des amides est développé dans le passage relatif à l'intermédiaire tétraédrique.

Lorsque la fonction électrophile du substrat risque de donner des réactions de type transestérification avec la base, il est alors souhaitable de choisir l'une des, ou les deux mesures suivantes, à savoir :
- que la basicité du groupe partant soit similaire ou supérieur à celle de la base mise initialement comme réactif.
- La voie consistant à utiliser le réactif de type Grignard.

Pour éviter certaines réactions parasites, il est souhaitable que ledit substrat ne soit pas acide ou présente un pKa au moins égal à 20, avantageusement à 25, de préférence à 30.

Le nombre global de carbone du substrat n'est pas limité sinon par la solubilité dans le milieu (avantageusement au moins égale à un dixième, de préférence une millimole par litre) et peut atteindre environ 50. Cependant il est préférable que l'on ne dépasse pas environ 30 atomes de carbone.

La réaction est en général menée à une température comprise entre la température de fusion du milieu et la température d'ébullition dans les conditions de pression de la réaction.

Plus spécifiquement, la réaction est menée en phase liquide, à une température comprise entre environ -100°C et 160°C, avantageusement entre environ - 60°C et 100°C. Lorsque R_{f}H est très volatil il est préférable pour s'assurer qu'il n'y ait pas d'évaporation ; pour ce faire il convient soit d'éviter que l'écart entre la température de réaction et le point d'ébulition soit trop important, plus précisement, il est souhaitable de travailler à une température qui ne soit pas supérieure au point d'ébultition (sous pression atmosphérique) de plus 100°C (deux chiffres significatifs), avantageusement de plus 80°C ; soit de travailler en enceinte fermée, soit d'opérer sous pression partielle élevée dudit R_{f}H. Soit de travailler en technique Grignard. Il est possible et même avantageux de combiner au moins deux des mesures ci-dessus.

Lorsque le substrat est sensible à la dégradation basique il est également préférable d'opérer à une température au plus égale à l'ambiante. S'il y a risque double de formation de carbène et sensibilité au base du substrat il est alors préférable de ne pas dépasser environ 10 C.

Selon la présente invention, la réaction est menée de manière à introduire en premier dans le milieu soit le matériau RfH, soit la base, et enfin le substrat. En d'autre terme de constituer un réactif à partir du matériau RfH, de la base et le cas échéant du solvant et/ou du diluant, puis de faire agir ce réactif sur le substrat. Cette variante est désignée ci-après sous l'expression de variante Grignard.

Dans les deux variantes ci-dessus, la réaction est menée de manière à introduire le dernier composant du réactif progressivement et avantageusement sur une période de temps comprise entre 5 et 300 minutes.

La réaction est menée de manière à introduire ladite base progressivement et avantageusement sur une période de temps comprise entre 5 et 300 minutes. (Cf. infra description du réactif).

En outre, il est souhaitable notamment lorsque la réaction est menée à des températures "élevées" (c'est-à-dire au moins égale à 240 K) que le rapport entre la quantité de matériau RfH et la base (RfH/base) soit au moins égal à une, avantageusement à 2 fois, et au plus égal à 10 ; de préférence au moins égal à 1 trois fois la quantité stoechiométrique et au plus égal à 5 fois.

Surtout et principalement si l'on procède selon la technique barbier, lorsque le substrat est sensible à la dégradation par base, il convient d'en limiter la quantité et surtout l'excès ; ainsi dans le cas où les substrats sont susceptibles de se dismuter, comme c'est le cas des aldéhydes pouvant donner lieu à des réactions de Cannizaro et/ou de Tishchenko, ou des réactions de crotonisation, il convient de limiter la quantité de base à 4/3, avantageusement à 5/4, de préférence à 1,1 la Q.S. (c'est-à-dire Quantité Stoechiométrique) par rapport au substrat.

L'utilisation de la procédure dite de Grignard, notamment en présence d'amide(s) (de préférence ceux visés comme précurseur du composé tétraédrique, cf. infra) résout pour l'essentiel le problème et permet ainsi d'utiliser de larges excès de base et donc de réactif. Alors un excès de 20 à 300 % est possible ; mais il est préférable de le limiter pour des questions de coût, en général, à une valeur d'environ 100 %. Bien entendu les mêmes valeurs sont applicables que lorsque le substrat est sensible aux bases.

Un autre but de la présente invention est de fournir un réactif qui puisse être utilisé pour la perfluoroalcoylation.

Ce but et d'autres qui apparaîtront par la suite sont atteints au moyen d'un réactif comportant un matériau de formule initiale RfH et une base (ou une espèce susceptible d'engendrer une base) dans un milieu polaire et anhydre.

Avantageusement le réactif comporte en outre au moins un solvant aprotique polaire.

En outre, il est souhaitable que le rapport entre la quantité de matériau RfH et la base (RfH/base) soit compris entre trois ets 1/2 fois la quantité stoechiométrique.

Ainsi qu'on l'a mentionné ci-dessus, le solvant joue un rôle important dans la présente invention et doit être aprotique, et avantageusement polaire et comporter très peu d'impuretés porteuses d'hydrogène acide.

Il est ainsi préférable que le solvant aprotique polaire utilisable ait un moment dipolaire significatif. Ainsi, sa constante diélectrique relative ε est avantageusement au moins égale à environ 5. De préférence l'e est inférieur ou égal à 50 (les zéros de position ne sont pas considérés comme des chiffres significatifs dans la présente description à moins qu'il en soit précisé autrement) et supérieur ou égal à 5.

On préfère en outre que les solvants de l'invention soient susceptibles de bien solvater les cations, ce qui peut être codifié par l'indice donneur D de ces solvants. Il est ainsi préférable que l'indice donneur D de ces solvants soit compris entre 10 et 30. Ledit indice donneur correspond au ΔH (variation d'Enthalpie), exprimé en kilocalorie, de l'association dudit solvant aprotique polaire avec le pentachlorure d'antimoine.

Selon la présente invention, il est préférable que le réactif ne présente pas d'hydrogène acide sur le ou les solvants polaires qu'il utilise. En particulier lorsque le caractère polaire du ou des solvants est obtenu par la présence de groupes électroattracteurs, il est souhaitable qu'il n'y ait pas d'hydrogène en alpha de la fonction électroattractrice.

De manière plus générale, il est préférable que le pKa correspondant à la première acidité du solvant soit au moins égal à environ 20 ("environ" soulignant que seul le premier chiffre est significatif), avantageusement au moins égal à 25, de préférence compris entre 25 et 35.

Il est préférable que ledit acide ou sel d'acide et ledit matériau soit au moins partiellement, de préférence complètement, soluble dans le milieu constituant le réactif.

Les solvants donnant de bons résultats peuvent être notamment des solvants de type amide. Parmi les amides, on comprend aussi les amides à caractère particulier comme les urées tétrasubstituées et les lactames monosubstitués. Les amides sont, de préférence, substitués (disubstitués pour les amides ordinaires). On peut citer par exemple les dérivés de pyrrolidone, tels que la N-méthylpyrrolidone, ou encore le N,N-diméthylformamide, ou le N,N-diméthyllacétamide.

Une autre catégorie particulièrement intéressante de solvants est constituée par les éthers, qu'il soient symétriques ou non symétriques, qu'ils soient ouverts ou non. Dans la catégorie des éthers doivent être incorporés les différents dérivés des éthers de glycol tels que les différents glymes, le diglyme par exemple.
Ainsi les solvants les plus adéquats, en raison de leurs prix et de leurs propriétés, sont avantageusement choisis parmi les éthers, notamment cycliques, tel le THF ou polyfonctionnels tels les glymes, ceux des amides qui, tels le DMF ou les DAAU (N,N'DiAlcoylAlcoylèneUrée) tels que la DMEU (N,N'DiMéthylEthylène-Urée) ou la DMPU (N,N'DiMéthylPropylèneUrée) n'ont pas d'hydrogène acide et les hétérocycles à caractère basique tels la pyridine. Lorsqu'ils sont employés, les amides utilisés jouent un rôle plus important qu'il n'y paraît de prime abord, et ils jouent un rôle dans la formation du réactif. En effet on a pu mettre en évidence que le réactif formé dans les amides (surtout lorsqu'ils répondent au précurseur de la formule du composé tétra édrique ci-après) était un réactif dont l'espèce réactive était le composé d'addition du CF₃⁻ sur le carbone de la fonction carbonyle, l'oxygène de cette dernière fonction devenant anionique.
C'est ce composé qui joue le rôle de vecteur de CF₃⁻ ou plus exactement de Rf⁻. Aussi une caractéristique importante de ce nouveau réactif est la présence de cette espèce dans le réactif. Aussi la présente invention vise aussi les réactifs contenant les composés de formule (IV) Rf-C[O⁻(M⁺)][R₁₃][N(R₁₁)(R₁₂)] 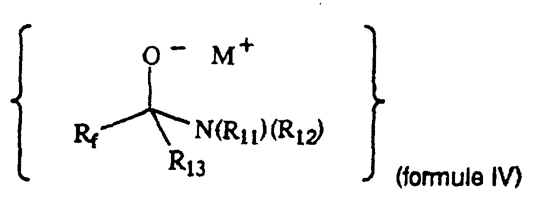 Bien sûr, la formule ci-dessus vise aussi l'autre énantiomère.
Cet intermédiaire est identifiable par RMN du fluor (dans le cas du diméthylformamide, δ de 1 ppm environ [doublet difficile à résoudre) par rapport à HCF₃).
Dans cette formule M⁺ représente un cation avantageusement monovalent et correspondant aux bases spécifiées dans la présente description ; avantageusement les alcalins et les phosphoniums.
Rf a déjà été défini ci dessus, R₁₁ ,R₁₂ et R₁₃ représentent des chaînes hydrocarbonées, aryle, y compris alcoylaryle, alcoyle, y compris aralcoyle et cycloalcoyle, ces chaînes peuvent être reliées entre elles pour former un (voire plusieurs) cycle(s). En ce qui concerne R₁₃ présente une valeur de constante de Hammett inférieure en valeur absolue à 0,2, de préférence à 0,1.

Toutefois R₁₃ peut aussi prendre la valeur hydrogène et c'est là sa valeur préférée. Une autre valeur satisfaisante de R₁₃ est la valeur aryle avantageusement dont la constante de Hammett est inférieure en valeur absolue à 0,2, de préférence à 0,1.
Cet intermédiaire présente une bonne stabilité, notamment aux basses températures (Par exemple -10, avantageusement -20, de préférence -30°C).
Ainsi selon la présente invention, vise un réactif du type précédent qui comporte au moins un composé de formule IV à une concentration au moins égale à une millimole par litre, avantageusement à 5 millimoles par litre, de préférence 10 millimoles par litre.

Cet intermédiaire peut servir de réactif de perfluoroalcoylation(s), comme décrit ci-dessus, mais aussi peut constituer un intermédiaire réactionnel conduisant des composés intéressants. En particulier aldéhyde, dérivé O-silylé, dérivé (bi)sulfitique , O-acylé.

Lorsqu'il est utilisé comme réactif de perfluoroalcoylation, il est préférable que les R₁₁ ,R₁₂ et R₁₃ soient de faible taille, c'est-à-dire que lorsqu'ils sont alcoyles leur nombre de carbones soit avantageusement au plus égal à 6, avantageusement à 3, de préférence méthyles; lorsqu'ils sont aryles, avantageusement phényles (substitués ou non), il est préférable que leur nombre de carbones soit avantageusement au plus égale à 10, avantageusement à 8. Il est préférable que globalement les R₁₁ ,R₁₂ et R₁₃ présente un nombre de carbones au plus égal à 15, avantageusement à 12, de préférence à 8.

Lorsqu'il n'est pas utilisé comme réactif de perfluoroalcoylation, mais comme intermédiaire de synthèse, les R₁₁ ,R₁₂ et R₁₃ peuvent être de taille plus élevée (sous réserve qu'il soit soluble dans le milieu), le nombre global de carbone peut alors atteindre environ 50. Cependant il est préférable que l'on ne dépasse pas environ 30 atomes de carbone.

Il est donc très recommandable en utilisant le réactif Grignard d'utiliser, seul(s) ou en mélange (éventuellement avec d'autres amides), les amides de formule R₁₃-CO-N(R₁₁)(R₁₂), le rapport préconisé entre ces amides et la base utilisée étant alors d'être au moins égal à 1, avantageusement à 2, de préférence à 5. Il n'y a pas de limite supérieure, sinon qu'il(s) constitue(nt) la totalité du solvant polaire. Quand ces amides sont utilisés comme solvants le plus souvent dans les tests effectués (sans que cela soit nécessairement un optimum) la teneur en ces amides par rapport à la somme des solvants polaires est comprise entre environ 40 et 80 %.

Parmi les diluants, on peut citer les hydrocarbures aliphatiques ou aromatiques, tels les alcanes ou les dérivés aryliques. Mention doit être faite des arylméthanes qui peuvent à la fois servir de diluant, (car inerte dans les conditions de la réaction) et de sources de base lorsque cette dernière est faite préalablement in situ.

Les exemples non limitatifs suivants illustrent l'invention.

### MODE OPERATOIRE TYPE:FLUOROFORME

### Méthode "Grignard"

A une solution * convenablement agitée de fluoroforme dans le DMF anhydre est additionnée goutte à goutte une base ou une solution de base dans un solvant polaire le plus souvent éther [cyclique ou non tel que THF éther symétrique ou non de dialcoyle (Par exemple éther de diméthyle, éther de di éthyle, éthers de dibutyle, éther de méthyle et d'éthyle,...), polyéther tel que les glymes] à - 40°C et sur une période de 10 min.

Le milieu réactionnel est laissé sans agitation 30 min. A -40°C avant d'additionner le substrat.

Celle solution est maintenue sans agitation à -40°C, 30 min. Supplémentaires avant l'addition d'acide acétique.

On laisse remonter la température à l'ambiante et la composition du mélange est déterminée par dosage CGL avec étalonnage interne.

### EXEMPLE N° 1 : ROLE DE L'ORDRE D'ADDITION DES REACTIFS

### ⇒ Mode opératoire général 2 (Méthode dite de "Grignard")

A une solution convenablement agitée (400t/min) de fluoroforme (3,0g, 43 mmol) dans 30 ml de DMF anhydre est additionnée goutte à goutte à -40°C sur une période 10 minutes, une solution de tBuOK 1M dans le THF (5 ml, 5mmol). Le milieu réactionnel est laissé sous agitation 30 minutes à -40°C, avant d'additionner le benzaldéhyde (0,47g, 4,4 mmol).

La solution est laissée sous agitation à -40°C, 30 minutes supplémentaires, avant addition d'acide acétique (0,5 ml).

La composition du mélange est déterminée par dosage CGL avec étalonnage interne:
TT(PhCHO) = 67%
RR(PhCHOHCF₃) = 46%
RR(PhCH₂OH) ⇒ traces

### EXEMPLE N° 2 (COMPARATIF) : ROLE DES REACTIONS PARASITES (REACTION DE CANNIZZARO SELON T.SHONO)

Dans la réaction entre le benzaldéhyde et le système CF₃H/t-BuOK/DMF, si les conditions opératoires ne sont pas biens choisies, les réactions parasites (et notamment formation d'alcool benzylique attribuée à la réaction de Cannizzaro par T. SHONO) sont majoritaires.
(mode opératoire général 1 (variation de la température réactionnelle et du nombre d'équivalent de base engagée).

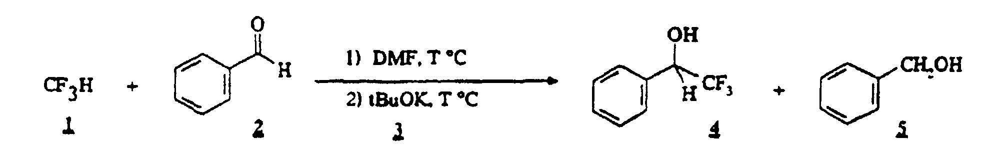

| Paramètres Expérimentaux | TT (2) % | RT (4) % | RT (5) % |
|---|---|---|---|
| Excès de base (2.2 éq.) à -50°C | 97 | 69 | - |
| Excès de base (1,5 éq.) en présence d'eau (20% mol /tBuOK engagé) à -50°C | 98 | 70 | 6 |
| Base (1 éq.) à -10°C | 88 | 73 | 3 |
| Excès de base (1,5 éq.) à -10°C | 100 | 19 | - |

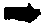 *un excès de base :* en présence d'un large excès de base (2,2 éq.) à -50°C, la formation d'alcool benzylique n'a pas lieu.
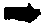 *La présence d'eau :* si l'un des réactifs est de mauvaise qualité, il peut contenir un peu d'eau qui induirait la réaction de Cannizzaro. Mais à -50°C, en présence d'eau (20% mol./t-BuOK engagé), on ne détecte guère que 6% d'alcool benzylique.
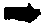 *Le niveau thermique* : à -10°C en présence d'un équivalent de base, la formation d'alcool benzylique n'a pas lieu ; en revanche la combinaison excès de base/niveau thermique (-10°C) favorise cette réaction puisque le rendement en trifluorométhylation chute de 70 à 19%.

### EXEMPLE N° 3 : ROLE DE LA NATURE DU SOLVANT

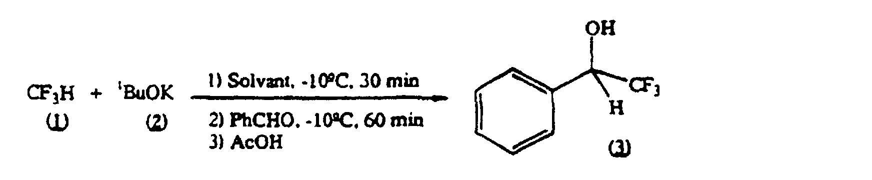

A une solution convenablement agitée (400t/min) de tBuOK (0,53g, 4,7 mmol) dans 30 ml de solvant anhydre(S) est additionnée, à -10°C, du fluoroforme (3g, 42,85 mmol). Le milieu réactionnel est laissé sous agitation 30 minutes à -10°C, avant d'additionner le benzaldéhyde (0,47g, 4,4 mmol).

La solution est laissée sous agitation à -10°C, 60 minutes supplémentaires, avant addition d'acide acétique (0,5 ml).

La composition du mélange est déterminée par dosage CGL avec étalonnage interne:

| Solvant | RR (3) % |
|---|---|
| THF | 25 |
| DMF | 57 |
| N-Formylpipéridine | 5 |

**EXEMPLE N° 4 : ROLE DE LA NATURE DE LA BASE**

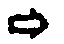 ***Cation associé* (Mode opératoire général de type 2)**

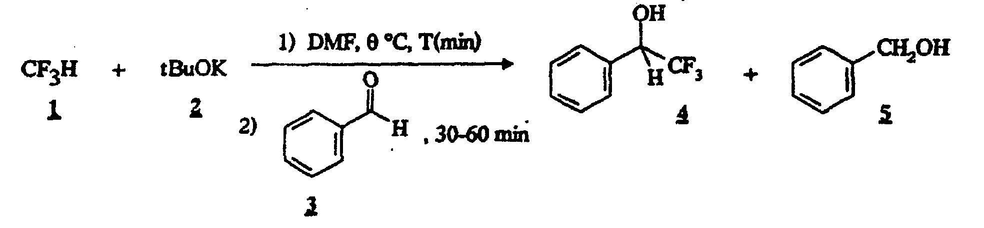

| tBuOM ^{*(a)*} | θ °C | T (min) | TT (3) (%) | RR (4) (%) | RT (4) (%) | RR (5) (%) | RT (5) (%) |
|---|---|---|---|---|---|---|---|
| tBuOK | -20 | 30 | 88 | 64 | 73 | traces | - |
| tBuONa | -20 | 30 | 83 | 59 | 71 | traces | - |
| tBuOLi | -20 | 30 | 32,5 | 13 | 40 | traces | - |

| | | | | | | | |
|---|---|---|---|---|---|---|---|
| *(a)* CF₃H/tBuOM/PhCOH (9/1,1/1). | | | | | | | |

**EXEMPLE N° 5 : MISE EN EVIDENCE ET ROLE DE L'INTERMEDIAIRE TETRAEDRIQUE**

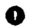 ***Synthèse d'hémiaminal du fluoral et de dérivés***

A une solution convenablement agitée de base dans 30 ml de DMF anhydre, on additionne à -10°C du fluoroforme (3g, 42,85 mmol). Cette solution est maintenue à -10°C durant 30 min puis on additionne goutte à goutte à cette même température:
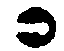 AcOH (0,37g, 6,2 mmol) dans le cas où R= H (base: KH/DMSO, 5,7 mmol),
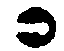 Me₃SiCl (1,3 ml, 10,25 mmol) dans le cas où R= Me₃Si (base: KHMDZ, 7 mmol)
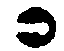 SO₂ (0,8g, 12,5 mmol) dans le cas où R= SO₂⁻K⁺ (base: KH/DMSO, 5,9 mmol).

Le milieu réactionnel est alors maintenu à cette même température durant 30 minutes avant de le laisser remonter à température ambiante.

Les produits formés ont été identifiés par RMN ¹H, ¹⁹F, ¹³C. 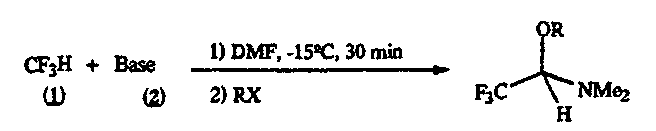
RX= AcOH, (3a), R=H
RX= Me₃SiCl, (3b), R= Me₃Si
RX=SO₂, (3c), R=SO₂⁻ K⁺

| RX | RR(dosé) |
|---|---|
| AcOH | 3a, 76% |
| Me₃SiCl | 3b, 79% |
| SO₂ | 3c, 77% |

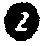 ***Synthèse d'hydrate de fluoral***

A une solution convenablement agitée de tBuOK (5 mmol) dans un solvant anhydre (30 ml), maintenue à -15°C, on ajoute du fluoroforme (3g, 42,85 mmol). Après 30 min à cette température, le milieu réactionnel est acidifié par 2 ml d'acide sulfurique.

Le tableau suivant donne les résultats en hydrate de fluoral en fonction des paramètres opératoires:

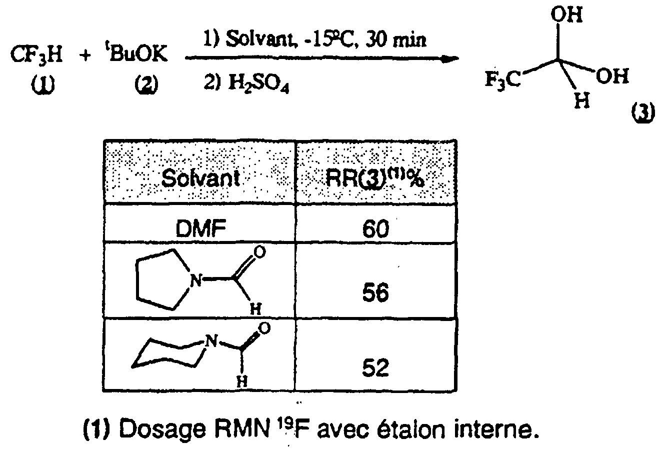

### EXEMPLE N° 6 : Synthèse de 2,2,2-trifluoroacetophénone

A une solution convenablement agitée (400t/min) de KHMDZ (1,15g, 5,75 mmol) dans 30 ml de DMF anhydre est additionnée à -10°C, du fluoroforme (3,0g, 43 mmol). Le milieu réactionnel est laissé sous agitation 30 minutes à -10°C, avant d'additionner goutte à goutte le benzoate de méthyle (0,51g, 3,75 mmol).

La solution est laissée sous agitation à -10°C, 1,5 heure supplémentaire, avant addition d'acide acétique (0,6 ml).

Après un traitement classique du milieu réactionnel (extraction et distillation), la trifluoroacétophénone est isolée avec un rendement de 55%.

### EXEMPLE N° 7 : 1,1,1,3,3,3-Hexafluoro-2-phényl-2-propanol

A une solution convenablement agitée (400t/min) de dimsylate de potassium (5,85 mmol) dans 30 ml d'un mélange DMF/DMSO anhydre (2/1), est additionné à -10°C, du fluoroforme (3,0g, 43 mmol). Le milieu réactionnel est laissé sous agitation 30 minutes à -10°C, avant d'additionner goutte à goutte la trifluoroacétophénone (0,615g, 3,5 mmol).

La solution est laissée sous agitation à -10°C, 1h10 supplémentaire, avant addition d'acide acétique (0,6 ml).

La composition du mélange est déterminée par dosage RMN 19F et CGL avec étalonnage interne:
TT(PhCOCF₃) = 35%
RR(PhCOH(CF₃)₂) = 79%
RT(PhCOH(CF₃)₂) = 44%

## Revendications

1. Procédé de synthèse organique de perfluoroalcoylation, **caractérisé par le fait qu'**il comporte une étape de mise en contact d'un matériau de formule RfH et d'une base, y compris une espèce susceptible d'engendrer une base, avec un substrat porteur d'au moins une fonction électrophile par addition dans un milieu polaire, **caractérisé par le fait que** la réaction est menée de manière à introduire en premier dans le milieu, soit le matériau RfH, soit la base et enfin le substrat en dernier.

2. Procédé selon la revendication 1, **caractérisé par le fait que** ledit matériau de formule RfH répond à la formule suivante :
H-(CX2)p-GEA
où les X semblables ou différents représentent un chlore, un fluor ou un radical de formule CₙF₂ₙ₊₁, avec n entier au plus égal à 5, de préférence à 2
où p représente un entier au plus égal à 2
où GEA représente un groupe électroattracteur dont les éventuelles fonctions sont inertes dans les conditions de la réaction, avantageusement fluor ou un reste perfluoré de formule CₙF₂ₙ₊₁, avec n entier au plus égal à 8, avantageusement à 5.

3. Procédé selon les revendications 1 et 2, **caractérisé par le fait que** ledit matériau de formule GEA répond à la formule III suivante :
R-CₙX'₂ₙ- (III)
où n est un entier au plus égal à 5,
où R est choisi parmi l'hydrogène, les alcoyles de 1 à 10 atomes de carbones, et les halogènes légers (chlore ou fluor, avantageusement fluor) ;
où les X', semblables ou différents, représentent un halogène léger (chlore ou fluor, avantageusement fluor) ou un radical de formule CₘF₂ₘ₊₁ avec m entier au plus égal à 5 de préférence à 2 .

4. Procédé selon les revendications 1 à 3, **caractérisé par le fait que** ladite base présente l'acide associé dont le pKa est au moins égal à 15, avantageusement à 20, de préférence à 30.

5. Procédé selon les revendications 1 à 4, **caractérisé par le fait que** ladite base présente un acide associé volatil dans les conditions de la réaction.

6. Procédé selon les revendications 1 à 5, **caractérisé par le fait que** ledit milieu polaire et anhydre est tel que l'acide le plus fort présent dans le milieu compte non tenu du matériau Rf et du substrat présente un pKa au moins égal à 25, avantageusement à 30, de préférence à 35.

7. Procédé selon les revendications 1 à 6, **caractérisé par le fait que** ledit milieu polaire et anhydre, y compris substrat et le matériau Rf, présente une quantité molaire en eau inférieure au quart de la quantité de base introduite, avantageusement au dixième, de préférence au cinquantième.

8. Procédé selon les revendications 1 à 7, **caractérisé par le fait que** ledit milieu polaire est anhydre, y compris substrat et matériau Rf, et présente une teneur molaire en eau au plus égale au tiers, avantageusement au quart, de préférence 1/10, par rapport à la quantité de base ajoutée.

9. Procédé selon les revendications 1 à 8, **caractérisé par le fait que** ledit milieu polaire et anhydre comporte au moins un solvant dont l'indice donneur est au moins égal à environ 10, avantageusement à environ 20, de préférence au plus égal à environ 30.

10. Procédé selon les revendications 1 à 9, **caractérisé par le fait que** ledit milieu polaire et anhydre comporte au moins un solvant dont la constante diélectrique relative est au moins égale à 5, avantageusement à 10, de préférence au plus égal à environ 50.

11. Procédé selon les revendications 1 à 10, **caractérisé par le fait que** ledit substrat est porteur d'au moins une fonction électrophile par addition.

12. Procédé selon les revendications 1 à 11, **caractérisé par le fait que** ledit substrat est porteur d'au moins une fonction électrophile par addition, choisie parmi les fonctions carbonyle, thiocarbonyle(>C=S), éventuellement conjuguées avec une ou plusieurs liaisons de type éthylénique, les chalcogènures portant un groupe partant et notamment les dichalcogènures, ont les chalcogènes sont de rang atomique au moins égal celui du soufre).

13. Procédé selon les revendications 1 à 12, **caractérisé par le fait que** ledit substrat présente un pKa au moins égal à 20, avantageusement à 25, de préférence à 30.

14. Procédé selon les revendications 1 à 13, **caractérisé par le fait que** la réaction est menée à une température comprise entre la température de fusion du milieu et la température d'ébullition dans les conditions de pression de la réaction.

15. Procédé selon les revendications 1 à 14, **caractérisé par le fait que** la réaction est menée à une température comprise entre - 50°C et 160°C.

16. Procédé selon les revendications 1 à 15, **caractérisé par le fait que** les cations associés à la base sont choisis parmi les alcalins et les phosphoniums quaternaires

17. Procédé selon les revendications 1 à 16, **caractérisé par le fait que** la réaction est menée de manière à introduire le dernier composant du réactif progressivement et avantageusement sur une période de temps comprise entre 5 et 300 minutes.

18. Procédé selon les revendications 1 à 17, **caractérisé par le fait que** la réaction est menée de manière à introduire ladite base progressivement et avantageusement sur une période de temps comprise entre 5 et 300 minutes.

19. Procédé selon les revendications 1 à 18, **caractérisé par le fait que** le rapport entre la quantité de matériau RfH et la base (RfH/base) est au moins égal à 1,5 fois la quantité stoechiométrique.

20. Réactif utile pour la perfluoroalcoylation selon les revendications 1 à 19, **caractérisé par le fait qu'**il comporte un matériau de formule RfH et une base, et que ledit matériau de formule RfH répond à la formule suivante :
H-(CX2)p-GEA
où les X, semblables ou différents, représentent un fluor ou un radical de formule CₙF₂ₙ₊₁, avec n entier au plus égal à 5, de préférence à 2 , voire un chlore ;
où p représente un entier au plus égal à 2 ;
où GEA représente un groupe électroattracteur dont les éventuelles fonctions sont inertes dans les conditions de la réaction, avantageusement fluor, ou un reste perfluoré de formule CₙF₂ₙ₊₁, avec n entier au plus égal à 8, avantageusement à 5 ;
et **par le fait que** le rapport entre la quantité de matériau RfH et la base est compris entre 3 et une demi-fois la quantité stoechiométrique.

21. Réactif selon la revendication 20, **caractérisé par le fait que** ladite base présente un acide associé présente un pKa au moins égal à 15, avantageusement à 20, de préférence à 30.

22. Réactif selon les revendications 20 et 21, **caractérisé par le fait que** ladite base présente un acide associé volatil dans les conditions de la réaction.

23. Réactif selon les revendications 20 à 22, **caractérisé par le fait que** ledit milieu polaire et anhydre est tel que l'acide le plus fort présent dans le milieu compte non tenu du matériau Rf et du substrat présente un pKa au moins égal à 25, avantageusement à 30, de préférence à 35.

24. Réactif selon les revendications 20 à 23, **caractérisé par le fait que** ledit milieu polaire et anhydre, y compris substrat et le matériau Rf, présente une quantité molaire en eau inférieure au quart de la quantité de base introduite, avantageusement au dixième, de préférence au cinquantième.

25. Réactif selon les revendications 20 à 24, **caractérisé par le fait que** ledit milieu polaire et anhydre comporte au moins un solvant dont l'indice donneur est au moins égal à environ 10, avantageusement à environ 20, de préférence au plus égal à environ 30.

26. Réactif selon les revendications 20 à 25, **caractérisé par le fait qu'**il comporte un composé de formule IV : 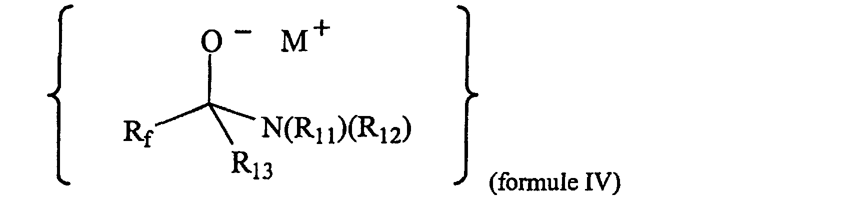 où M⁺ représente un cation monovalent; avantageusement les alcalins et les phosphoniums quaternaires (avantageusement d'au plus 40 atomes de carbone, de préférence d'au plus 24).
R₁₁, R₁₂ et R₁₃ représentent des chaînes hydrocarbonées telles que aryle, y compris alcoylaryle, alcoyle, y compris aralcoyle et cycloalcoyle. Ces chaînes peuvent être reliées entre elles pour former un (voire plusieurs) cycle(s).

27. Réactif selon les revendications 20 à 26, **caractérisé par le fait que** la concentration en composé de formule IV est au moins égale à une millimole par litre.

## Patentansprüche

1. Verfahren der organischen Synthese zur Perfluoralkylierung, **dadurch gekennzeichnet, daß** es eine Stufe des In-Kontakt-Bringens eines Materials der Formel RfH und einer Base, einschließlich eines Stoffes, der geeignet ist, eine Base zu erzeugen, mit einem Substrat, das mindestens eine elektrophile Funktion trägt, durch Addition in einem polaren Medium umfaßt, und **dadurch gekennzeichnet, daß** die Reaktion in der Weise geführt wird, daß zuerst in das Medium entweder das Material RfH oder die Base und zuletzt das Substrat eingetragen werden.

2. Verfahren nach Anspruch 1, **dadurch gekennzeichnet, daß** das genannte Material der Formel RfH der folgenden Formel entspricht:
H-(CX₂)p-GEA
worin die Symbole X, gleich oder verschieden, ein Chlor, ein Fluor oder einen Rest der Formel CₙF₂ₙ₊₁ darstellen, mit n einer ganzen Zahl von höchstens 5, vorzugsweise von 2,
worin p eine ganze Zahl von höchstens 2 ist,
worin GEA eine elektroattraktive Gruppe, deren eventuelle Funktionen unter den Bedingungen der Reaktion inert sind, darstellt, vorzugsweise Fluor oder einen perfluorierten Rest der Formel CₙF₂ₙ₊₁ mit n einer ganzen Zahl von höchstens 8, vorteilhafterweise 5.

3. Verfahren nach Anspruch 1 und 2, **dadurch gekennzeichnet, daß** das genannte Material der Formel GEA der folgenden Formel (III) entspricht:
R-CₙX'₂ₙ₋ (III)
worin n eine ganze Zahl von höchstens 5 ist,
worin R unter Wasserstoff, den Alkylen mit 1 bis 10 Kohlenstoffatomen und den leichten Halogenen (Chlor oder Fluor, vorteilhafterweise Fluor) ausgewählt wird,
worin die Symbole X', gleich oder verschieden, ein leichtes Halogen (Chlor oder Fluor, vorteilhafterweise Fluor) oder einen Rest der Formel CₘF₂ₘ₊₁ darstellen, mit m einer ganzen Zahl von höchstens 5, vorzugsweise 2.

4. Verfahren nach Anspruch 1 bis 3, **dadurch gekennzeichnet, daß** die genannte Base eine assoziierte Säure aufweist, deren pKa mindestens gleich 15, vorteilhafterweise 20 und vorzugsweise 30 beträgt.

5. Verfahren nach Anspruch 1 bis 4, **dadurch gekennzeichnet, daß** die genannte Base eine assoziierte Säure aufweist, die unter den Bedingungen der Reaktion flüchtig ist.

6. Verfahren nach Anspruch 1 bis 5, **dadurch gekennzeichnet, daß** das genannte wasserfreie und polare Medium so beschaffen ist, daß die stärkste in dem Medium anwesende Säure, nicht unter Berücksichtigung des Materials Rf und des Substrates, einen pKa von mindestens gleich 25, vorteilhafterweise von 30 und vorzugsweise von 35 aufweist.

7. Verfahren nach Anspruch 1 bis 6, **dadurch gekennzeichnet, daß** das genannte wasserfreie und polare Medium, einschließlich des Substrates und des Materials Rf, eine molare Menge an Wasser von unter einem Viertel der Menge der eingetragenen Base aufweist, vorteilhafterweise von einem Zehntel, vorzugsweise von einem Fünfzigstel.

8. Verfahren nach Anspruch 1 bis 7, **dadurch gekennzeichnet, daß** das genannte wasserfreie und polare Medium, einschließlich des Substrates und des Materials Rf, einen molaren Gehalt an Wasser von höchstens gleich einem Drittel aufweist, vorteilhafterweise von einem Viertel, vorzugsweise von einem Zehntel, bezogen auf die Menge der zugesetzten Base.

9. Verfahren nach Anspruch 1 bis 8, **dadurch gekennzeichnet, daß** das genannte wasserfreie und polare Medium mindestens ein Lösungsmittel umfaßt, dessen Donator-Index mindestens gleich etwa 10 beträgt, vorteilhafterweise etwa 20 und vorzugsweise höchstens gleich etwa 30.

10. Verfahren nach Anspruch 1 bis 9, **dadurch gekennzeichnet, daß** das genannte wasserfreie und polare Medium mindestens ein Lösungsmittel umfaßt, dessen relative Dielektrizitätskonstante mindestens gleich 5 beträgt, vorteilhafterweise 10 und vorzugsweise höchstens gleich etwa 50.

11. Verfahren nach Anspruch 1 bis 10, **dadurch gekennzeichnet, daß** das genannte Substrat Träger von mindestens einer elektrophilen Funktion durch Addition ist.

12. Verfahren nach Anspruch 1 bis 11, **dadurch gekennzeichnet, daß** das genannte Substrat Träger von mindestens einer elektrophilen Funktion durch Addition ist, ausgewählt unter den Funktionen Carbonyl, Thiocarbonyl (>C=S), gegebenenfalls konjugiert mit einer oder mehreren Bindungen vom ethylenischen Typ, den Chalkogeniden, die eine Abgangsgruppe tragen und insbesondere den Dichalkogeniden, wobei die Chalkogene einen Atomrang von mindestens dem des Schwefels besitzen.

13. Verfahren nach Anspruch 1 bis 12, **dadurch gekennzeichnet, daß** das genannte Substrat einen pKa von mindestens gleich 20 aufweist, vorteilhafterweise von 25, vorzugsweise von 30.

14. Verfahren nach Anspruch 1 bis 13, **dadurch gekennzeichnet, daß** die Reaktion bei einer Temperatur geführt wird, die unter den Druckbedingungen der Reaktion zwischen der Schmelztemperatur des Mediums und der Siedetemperatur liegt.

15. Verfahren nach Anspruch 1 bis 14, **dadurch gekennzeichnet, daß** die Reaktion bei einer Temperatur zwischen -50 °C und 160 °C durchgeführt wird.

16. Verfahren nach Anspruch 1 bis 15, **dadurch gekennzeichnet, daß** die mit der Base assoziierten Kationen unter den Alkalien und den quaternären Phosphonium-Verbindungen ausgewählt werden.

17. Verfahren nach Anspruch 1 bis 16, **dadurch gekennzeichnet, daß** die Reaktion in der Weise geführt wird, daß der letzte Bestandteil des Reaktanden nach und nach und vorteilhafterweise über eine Zeitdauer zwischen 5 und 300 Minuten eingetragen wird.

18. Verfahren nach Anspruch 1 bis 17, **dadurch gekennzeichnet, daß** die Reaktion in der Weise geführt wird, daß die genannte Base nach und nach und vorteilhafterweise über eine Zeitdauer zwischen 5 und 300 Minuten eingetragen wird.

19. Verfahren nach Anspruch 1 bis 18, **dadurch gekennzeichnet, daß** das Verhältnis zwischen der Menge des Materials RfH und der Base (RfH/Base) mindestens gleich 1,5-mal der stöchiometrischen Menge beträgt.

20. Reagens, geeignet für die Perfluoralkylierung nach den Ansprüchen 1 bis 19, **dadurch gekennzeichnet, daß** es ein Material der Formel RfH und eine Base umfaßt, und daß das genannte Material der Formel RfH der folgenden Formel entspricht:
H-(CX₂)p-GEA
worin die Symbole X, gleich oder verschieden, ein Fluor oder einen Rest der Formel CₙF₂ₙ₊₁ darstellen, mit n einer ganzen Zahl von höchstens 5, vorzugsweise von 2,
worin p eine ganze Zahl von höchstens 2 ist,
worin GEA eine elektroattraktive Gruppe, deren eventuelle Funktionen unter den Bedingungen der Reaktion inert sind, darstellt, vorzugsweise Fluor oder einen perfluorierten Rest der Formel CₙF₂ₙ₊₁ mit n einer ganzen Zahl von höchstens 8, vorteilhafterweise von 5;
und dadurch, daß das Verhältnis zwischen der Menge des Materials RfH und der Base zwischen der dreifachen und der halben stöchiometrischen Menge beträgt.

21. Reagens nach Anspruch 20, **dadurch gekennzeichnet, daß** die genannte Base eine assoziierte Säure aufweist, deren pKa mindestens gleich 15, vorteilhafterweise 20 und vorzugsweise 30 beträgt.

22. Reagens nach Anspruch 20 und 21, **dadurch gekennzeichnet, daß** die genannte Base eine assoziierte Säure aufweist, die unter den Bedingungen der Reaktion flüchtig ist.

23. Reagens nach Anspruch 20 bis 22, **dadurch gekennzeichnet, daß** das genannte wasserfreie und polare Medium so beschaffen ist, daß die stärkste in dem Medium anwesende Säure, nicht unter Berücksichtigung des Materials Rf und des Substrates, einen pKa von mindestens gleich 25, vorteilhafterweise von 30 und vorzugsweise von 35 aufweist.

24. Reagens nach Anspruch 20 bis 23, **dadurch gekennzeichnet, daß** das genannte wasserfreie und polare Medium, einschließlich des Substrates und des Materials Rf, eine molare Menge an Wasser von unter einem Viertel der Menge der eingetragenen Base aufweist, vorteilhafterweise von einem Zehntel, vorzugsweise von einem Fünfzigstel.

25. Reagens nach Anspruch 20 bis 24, **dadurch gekennzeichnet, daß** das genannte wasserfreie und polare Medium mindestens ein Lösungsmittel umfaßt, dessen Donator-Index mindestens gleich etwa 10 beträgt, vorteilhafterweise etwa 20 und vorzugsweise höchstens gleich etwa 30.

26. Reagens nach Anspruch 20 bis 25, **dadurch gekennzeichnet, daß** es eine Verbindung der Formel (IV) umfaßt: 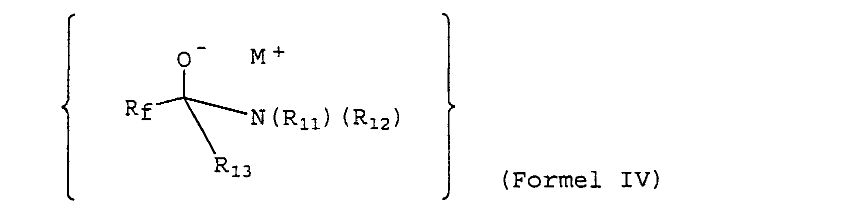 worin M⁺ ein monovalentes Kation darstellt, vorteilhafterweise die Alkalien und die quaternären Phosphonium-Verbindungen (vorteilhafterweise mit höchstens 40 Kohlenstoffatomen, vorzugsweise höchstens 24 Kohlenstoffatomen),
R₁₁, R₁₂ und R₁₃ Kohlenwasserstoff-Ketten darstellen wie Aryl, einschließlich Alkylaryl, Alkyl, einschließlich Aralkyl und Cycloalkyl, wobei diese Ketten untereinander verbunden sein können, um einen (sogar mehrere) Ring(e) zu bilden.

27. Reagens nach Anspruch 20 bis 26, **dadurch gekennzeichnet, daß** die Konzentration an der Verbindung der Formel (IV) mindestens gleich einem Millimol pro Liter beträgt.

## Claims

1. Organic synthesis process for perfluoroalkylation, **characterized in that** it comprises a stage in which a material of formula RfH and a base, including a species capable of generating a base, are brought into contact with a substrate carrying at least one electrophilic functional group by addition to a polar medium, **characterized in that** the reaction is carried out so as to introduce firstly into the medium either the material RfH or the base, and finally the substrate last.

2. Process according to Claim 1, **characterized in that** the said material of formula RfH corresponds to the following formula:
H-(CX2)p-EWG
where the X, which are alike or different, represent a chlorine, a fluorine or a radical of formula CₙF₂ₙ+₁ with n an integer at most equal to 5, preferably to 2
where p represents an integer at most equal to 2
where EWG represents an electron-withdrawing group, the possible functional groups of which are inert under the reaction conditions, advantageously fluorine or a perfluoro residue of formula CₙF₂ₙ₊₁ with n an integer at most equal to 8, advantageously to 5.

3. Process according to Claims 1 and 2, **characterized in that** the said material of formula EWG corresponds to the following formula III:
R-CₙX'₂ₙ- (III)
where n is an integer at most equal to 5,
where R is chosen from hydrogen, alkyls containing 1 to 10 carbon atoms and light halogens (chlorine or fluorine, advantageously fluorine) ;
where the X', which are alike or different, represent a light halogen (chlorine or fluorine, advantageously fluorine) or a radical of formula CₘF₂ₘ₊₁ with m an integer at most equal to 5, preferably to 2.

4. Process according to Claims 1 to 3, **characterized in that** the said base has the associated acid, the pKa of which is at least equal to 15, advantageously to 20, preferably to 30.

5. Process according to Claims 1 to 4, **characterized in that** the said base has an associated acid which is volatile under the reaction conditions.

6. Process according to Claims 1 to 5, **characterized in that** the said polar and anhydrous medium is such that the strongest acid present in the medium, not taking into account the material Rf and the substrate, has a pKₐ at least equal to 25, advantageously to 30, preferably to 35.

7. Process according to Claims 1 to 6, **characterized in that** the said polar and anhydrous medium, including substrate and the material Rf, exhibits a molar amount of water of less than a quarter of the amount of base introduced, advantageously than a tenth, than a fiftieth.

8. Process according to Claims 1 to 7, **characterized in that** the said polar medium is anhydrous, including substrate and material Rf, and exhibits a molar content of water at most equal to a third, advantageously to a quarter, preferably 1/10, with respect to the amount of base added.

9. Process according to Claims 1 to 8, **characterized in that** the said polar and anhydrous medium contains at least one solvent with a donor number at least equal to approximately 10, advantageously to approximately 20, preferably at most equal to approximately 30.

10. Process according to Claims 1 to 9, **characterized in that** the said polar and anhydrous medium contains at least one solvent with a relative dielectric constant at least equal to 5, advantageously to 10, preferably at most equal to approximately 50.

11. Process according to Claims 1 to 10, **characterized in that** the said substrate carries at least one electrophilic functional group by addition.

12. Process according to Claims 1 to 11, **characterized in that** the said substrate carries at least one electrophilic functional group by addition chosen from carbonyl or thiocarbonyl(>C=S) functional groups, optionally conjugated with one or more bonds of ethylene type, chalcogenides carrying a leaving group and in particular dichalcogenides, have the chalcogens have atomic rank at least equal [lacuna] that of sulphur).

13. Process according to Claims 1 to 12, **characterized in that** the said substrate has a pKa at least equal to 20, advantageously to 25, preferably to 30.

14. Process according to Claims 1 to 13, **characterized in that** the reaction is carried out at a temperature of between the melting temperature of the medium and the boiling temperature under the pressure conditions of the reaction.

15. Process according to Claims 1 to 14, **characterized in that** the reaction is carried out at a temperature of between -50°C and 160°C.

16. Process according to Claims 1 to 15, **characterized in that** the cations associated with the base are chosen from alkali metals and quaternary phosphoniums.

17. Process according to Claims 1 to 16, **characterized in that** the reaction is carried out so as to introduce the final component of the reactant gradually and advantageously over a period of time of between 5 and 300 minutes.

18. Process according to Claims 1 to 17, **characterized in that** the reaction is carried out so as to introduce the said base gradually and advantageously over a period of time of between 5 and 300 minutes.

19. Process according to Claims 1 to 18, **characterized in that** the ratio of the amount of material RfH to the base (RfH/base) is at least equal to 1.5 times the stoichiometric amount.

20. Reactant of use in perfluoroalkylation according to Claims 1 to 19, **characterized in that** it contains a material of formula RfH and a base and **in that** the said material of formula RfH corresponds to the following formula:
H-(CX2)p-EWG
where the X, which are alike or different, represent a fluorine or a radical of formula CₙF₂ₙ₊₁ with n an integer at most equal to 5, preferably to 2, or a chlorine;
where p represents an integer at most equal to 2;
where EWG represents an electron-withdrawing group, the possible functional groups of which are inert under the reaction conditions, advantageously fluorine or a perfluoro residue of formula CₙF₂ₙ₊₁ with n an integer at most equal to 8, advantageously to 5; and **in that** the ratio between the amount of material RfH and the base is between 3 and one-half of the stoichiometric amount.

21. Reactant according to Claim 20, **characterized in that** the said base has an associated acid [lacuna] has a pKa at least equal to 15, advantageously to 20, preferably to 30.

22. Reactant according to Claims 20 and 21, **characterized in that** the said base has an associated acid which is volatile under the reaction conditions.

23. Reactant according to Claims 20 to 22, **characterized in that** the said polar and anhydrous medium is such that the strongest acid present in the medium, not taking into account the material Rf and the substrate, has a pKₐ at least equal to 25, advantageously to 30, preferably to 35.

24. Reactant according to Claims 20 to 23, **characterized in that** the said polar and anhydrous medium, including substrate and the material Rf, has a molar amount of water of less than a quarter of the amount of base introduced, advantageously than a tenth, preferably than a fiftieth.

25. Reactant according to Claims 20 to 24, **characterized in that** the said polar and anhydrous medium contains at least one solvent, the donor number of which is at least equal to approximately 10, advantageously to approximately 20, preferably at most equal to approximately 30.

26. Reactant accoring to Claims 20 to 25, **characterized in that** it comprises a compound of formula IV: 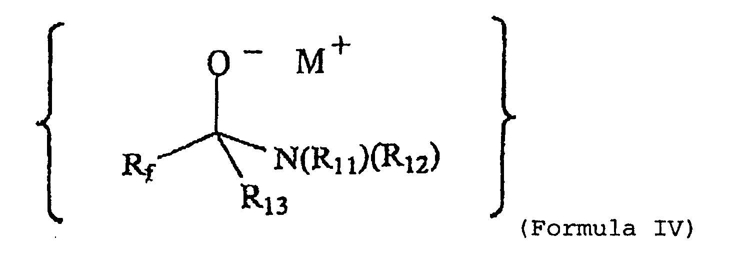 where M⁺ represents a monovalent cation, advantageously alkali metals and quaternary phosphoniums (advantageously of at most 40 carbon atoms, preferably of at most 24),
R₁₁, R₁₂ and R₁₃ represent hydrocarbon-comprising chains, such as aryl chains, including alkylaryl or alkyl chains, including aralkyl and cycloalkyl chains. These chains can be connected to one another in order to form one (or more) ring(s).

27. Reagent according to Claims 20 to 26, **characterized in that** the concentration of compound of formula IV is at least equal to one millimole per litre.
